# EUROPEAN PATENT SPECIFICATION

(11) **EP 0 647 142 B1**
(45) Date of publication and mention of the grant of the patent: **16.09.1998**
(21) Application number: 93915313.6
(22) Date of filing: 14.06.1993
(51) Int. Cl.: A61L 15/24, A61L 15/62

(54) **BIODEGRADABLE, LIQUID IMPERVIOUS MONOLAYER FILM COMPOSITIONS**
ZUSAMMENSETZUNGEN FÜR BIODEGRADIERBARE FLÜSSIGKEITSUNDURCHLÄSSIGE EINZELSCHICHTEN
COMPOSITIONS DE FILMS MONOCOUCHES BIODEGRADABLES ET IMPERMEABLES AUX LIQUIDES

(30) Priority: 26.06.1992 US 904776
(43) Date of publication of application: 12.04.1995
(73) Proprietor: THE PROCTER & GAMBLE COMPANY, Cincinnati, Ohio 45202 (US)
(72) Inventor: WNUK, Andrew, Julian, Wyoming, OH 45215 (US); MELIK, David, Harry, Fairfield, OH 45014 (US); YOUNG, Terrill Alan, Cincinnati, OH 45239 (US)
(74) Representative: Bottema, Johan Jan
(86) International application number: US9305618
(87) International publication number: WO9400163

(56) References cited:
- EP-A- 0 226 439
- EP-A- 0 435 435
- EP-A- 0 450 777
- WO-A-92/01733
- WO-A-92/04410
- GB-A- 2 243 327
- US-A- 4 962 164
- US-A- 5 026 589
- DATABASE WPIL Week 9232, Derwent Publications Ltd., London, GB; AN 92-263106 & JP,A,4 178 466 (SHINGIJUTSU KAIHATSU JIGYODAN) 25 June 1992
- DATABASE WPIL Week 9219, Derwent Publications Ltd., London, GB; AN 92-155471 & JP,A,4 093 315 (DENKI KAGAKU KOGYO) 10 August 1990

## Description

### TECHNICAL FIELD

The present invention relates to liquid impervious films comprising blends of biodegradable thermoplastic polymers. The films are especially suitable for use as backsheets in articles such as diapers, sanitary napkins, pantiliners, and the like, which are adapted for absorbing various bodily fluids. The films herein may also be used in a sealable packaging film, plastic garbage bags, etc.

### BACKGROUND OF THE INVENTION

A wide variety of absorbent articles designed to be efficient for the absorption of body fluids such as blood, urine, menses, and the like, are known. Disposable products of this type generally comprise some sort of fluid-permeable topsheet material, an absorbent core, and a fluid-impermeable backsheet material. Heretofore, such absorbent structures have been prepared using, for example, topsheet materials prepared from woven, nonwoven, or porous formed-film polyethylene or polypropylene materials. Backsheet materials typically comprise flexible polyethylene sheets. Absorbent core materials typically comprise wood pulp fibers or wood pulp fibers in combination with absorbent gelling materials. One aspect of such absorbent articles that has recently been considered is their disposability. Although such products largely comprise materials which would be expected ultimately to degrade, and although products of this type contribute only a very small percentage of the total solid waste materials generated by consumers each year, nevertheless, there is currently a perceived need to devise such disposable products from materials which are compostable.

A conventional disposable absorbent product is already to a large extent compostable. A typical disposable diaper, for example, consists of about 80% of compostable materials, e.g., wood pulp fibers, and the like. In the composting process soiled disposable absorbent articles are shredded and commingled with organic waste prior to the composting per se. After composting is complete, the non-compostable particles are screened out. In this manner even today's absorbent articles can successfully be processed in commercial composting plants.

Nevertheless, there is a need for reducing the amount of non-compostable materials in disposable absorbent articles. There is a particular need to replace polyethylene backsheets in absorbent articles with liquid impervious films of compostable material, because the backsheet is typically one of the largest non-compostable components of a conventional disposable absorbent article.

In addition to being compostable, the films employed as backsheets for absorbent articles must satisfy many other performance requirements. For example, the resins should be thermoplastic such that conventional film processing methods can be employed. These methods include cast film and blown film extrusion of single layer structures and cast or blown film coextrusion of multilayer structures. Other methods include extrusion coating of one material on one or both sides of a compostable substrate such as another film, a non-woven fabric, or a paper web.

Still other properties are essential in product converting operations where the films are used to fabricate absorbent articles. Properties such as tensile strength, tensile modulus, tear strength, and thermal softening point determine to a large extent how well a film will run on converting lines.

In addition to the aforementioned properties, still other properties are needed to meet the end user requirements of the absorbent article. Film properties such as impact strength, puncture strength, and moisture transmission are important since they influence the absorbent article's durability and containment while being worn.

Once the absorbent article is disposed of and enters a composting process, other properties become important. Regardless of whether incoming waste is preshredded or not, it it important that the film or large film fragments undergo an initial breakup to much smaller particles during the initial stages of composting. Otherwise, the films or large fragments may be screened out of the compost stream and may never become part of the final compost.

During the initial stages of composting, for example where a Daneco drum is employed, the film is exposed to mechanical action, elevated temperatures, and moisture, in addition to microorganisms. Any one or two or all three of these elements can be used to promote the initial breakup of the film or large film fragments to much smaller fragments.

Many biodegradable polymers exist which are sensitive to mechanical action or elevated temperatures, or moisture. Many would individually meet the requirements for composting. However, few if any, can meet all processing, converting, end user, and disposal requirements of films suitable for backsheets of absorbent articles. To meet all these requirements simultaneously in a single film, various biodegradable polymers must be combined in ways which overcome their individual deficiencies but do not compromise the beneficial properties associated with the individual biodegradable polymers.

It is, therefore, an object of the present invention to provide a monolayer backsheet film having
a) biodegradability;
b) liquid-impermeability;
c) a machine direction (MD) tensile modulus from 6.895 x 10⁸ dynes/sq. cm. to 6.895 x 10⁹ dynes/sq. cm.;
d) a MD tear strength of at least 70 grams per 25,4 microns of thickness;
e) a cross machine direction (CD) tear strength of at least 70 grams per 25.4 microns of thickness;
f) an impact strength of at least 12 centimeters as measured by falling ball drop;
g) a moisture transport rate less than 0.0012 grams per square centimeter per 16 hours;
h) a modulus at 60°C of at least 5.52 x 10⁷ dynes/sq. cm.; and
i) a thickness from 12 microns to 75 microns;
said film comprising two or more polymers selected from the group consisting of:
a) moisture sensitive polymers, preferably thermoplastic polyvinyl alcohol compositions or hydroxypropylcellulose;
b) thermally sensitive polymers, preferably polycaprolactone, other aliphatic polyesters having melting points below 65°C or Vicat softening points of 45°C or less, or mixtures thereof;
c) mechanically limited polymers, preferably cellulose esters, cellulose ester blends with polycaprolactone, cellulose ester blends with polyhydroxybutyrate/valerate copolymers, or cellulose ester blends with thermally sensitive aliphatic polyesters;
d) polymers difficult to process into films, preferably polyhydroxybutyrate, polyhydroxybutyrate/valerate copolymers, or mixtures thereof;
e) hydrolytically cleavable aromatic/aliphatic polyester copolymers, preferably copolymers of polyethylene terephthalate or polybutylene terephthalate wherein the aliphatic fraction of said copolymers is derived from the group consisting of oxalates, malonates, succinates, glutarates, adipates, pimelates, suberates, azelotes, sobacates, nonanedinates, glycolates, and mixtures thereof;
f) oxidized ethylene/carbon monoxide copolymers, preferably derived from ethylene/monoxide copolymers containing 0.5 to 20 weight percent carbon monoxide;
g) high melting aliphatic polyesters; preferably aliphatic polyesters having melting temperatures or glass transition temperatures above 65°C; and
h) elastomers, preferably polycaprolactone/diene block copolymers or aliphatic polyester urethanes,
said film being characterized by comprising either
- 1-40 weight percent of a moisture sensitive polymer, preferably a thermoplastic polyvinyl alcohol composition; and
- 99-60 weight percent of a second polymer selected from the group consisting of hydrolytically cleavable aromatic/aliphatic polyester copolymers, oxidized ethylene/carbon monoxide copolymers, and high melting aliphatic polyesters;
or said film being characterized by comprising
- 30-70 weight percent of a mechanically limited polymer, preferably a cellulose ester; and
- 70-30 weight percent of an elastomer, preferably an aliphatic polyester urethane.

### BACKGROUND ART

Degradable mulch films comprising starch, polyvinylalcohol, and glycerol are disclosed in U.S. Patent 3,939,145 issued to Otey and Mark on April 6, 1976. The degradability of the films is controlled by application of a non-degradable water resistant coating based on mixtures of polyol-toluene diisocyanate prepolymers with poly (vinylidene chloride/acrylonitrile) copolymers or poly(vinyl chloride) resins mixed with plasticizers.

Degradable mulch films with improved moisture resistance and comprising starch and ethylene/acrylic acid copolymers are disclosed in U.S. Patent 4,133,784 issued to Otey and Westhoff on January 9, 1979 and U.S. Patent 4,337,181 issued to Otey and Westhoff on June 29, 1982. The films disclosed in the latter patent also include a neutralizing agent such as ammonia, which allows them to be processed by blown film technology with good properties. Polyethylene is specified as an optional component in the films. Applications such as garbage bags and various types of packaging are also contemplated in U.S. Patent 4,337,181.

International Patent Application WO90/10671 discloses biodegradable articles based on starch. In particular, destructurized starch and ethylene/acrylic acid copolymer are combined to form an interpenetrated network. It has also been disclosed that an ethylene/vinyl alcohol copolymer can be used in place of the ethylene/acrylic acid copolymer. Procedures for extruding sheets and films are also disclosed.

European Patent Application 89810373.4 discloses polymer compositions obtained from a melt comprising at least one water-containing destructurized hydrophilic polymer and at least one synthetic essentially water-insoluble thermoplastic polymer. The materials are said to be shapable under conditions of controlled water content and pressure into bottles, sheets, films, packaging materials, sacks, bags, and other articles.

U.S. Patent 5,095,054 issued to Lay et al. on March 10, 1992 discloses thermoplastic polymer compositions comprising a (1) destructurized starch component, (2) a second component selected from one of twelve groups of polymers bearing various types of chemically functional moieties, or (3) at least one substantially water-insoluble thermoplastic polymer. Combinations of (1), (2), and (3) are also disclosed.

A biodegradable, liquid impervious sheet laminate is described in British Patent Application GB 2,243,327 A. The laminate comprises a layer of biodegradable thermoplastic polymer, preferably copolymers of hydroxybutyric acid and other hydroxy acids, and at least one layer of a biodegradable woven or non-woven fibrous substrate, preferably a rayon nonwoven fabric. The tensile strength of sheet laminates suitable for backsheets of absorbent articles such as diapers, is at least 4 Newtons, preferably at least 5 Newtons.

Disposable sanitary articles comprising biodegradable film and fiber components derived from polymers based on dioxanone are disclosed in U.S. Patent 5,026,589 issued to Schechtman on June 25, 1991. Also disclosed are copolymers of dioxanones with other monomers such as caprolactone, lactic acid, glycolic acid, and the like. Specific copolymer compositions providing properties essential to backsheet use are not given.

U.S. Patent 4,873,270 discloses a substance based on polyurethane from which biodegradable materials can be obtained by rolling or calendering. The substance comprises a homogeneous mixture of a thermoplastic polyurethane resin, a carbohydrate, a second thermoplastic resin to make the substance calenderable which comprises polyvinyl chloride (PVC), and an additional biodegradable resin which is a biodegradable aliphatic polyester, preferably of the same nature as the polyurethane resin. For example, the substance could contain a polycaprolactone-based polyurethane, starch, PVC, and polycaprolactone.

International Patent application WO 91/13207 discloses biodegradable sheet structures based on paper substrates coated with a latex and subsequently dried. The latex comprises a colloidal suspension in water of essentially non-crystalline particles of a biodegradable polymer such as polyhydroxybutyrate or polyhydroxybutyrate/valerate copolymer.

European Patent Application EP-449041 discloses non-thermoplastic blends of cellulose hydrate with urea-based polyurethanes prepared by mixing viscose with the polyurethane, gelling the mixture at above 40°C with an acidic coagulant and regenerating agent, treating the gel with plasticizers, and finally drying the film. The films are said to be useful as packaging films, refuse sacks, mulch films, diaper covers, and the like.

U.S. Patent Application 5,037,410 discloses diaper backsheets composed of 20-80% by weight unvulcanized C4-C6 diene elastomer, for example natural rubber, 5-20% modifier such as starch, 1-40% inorganic fillers, 0-40% styrene-based resin, plus waxes, and other ingredients. The backsheets are reported to have tensile strengths above 200 grams per inch of width.

European Patent Application 91106627.2 discloses a biodegradable latex web material suitable for diaper backsheets and mulch films. The materials are prepared by impregnating a cellulose fiber or paper web with a carboxylated latex comprising both synthetic polymers such as styrene-butadiene rubber and PVC, and natural polymers such as starch, proteins and natural rubber. Tensile strengths ranging from 189 to 304 kg/sq.cm. are cited.

U.S. Patent 3,952,347 discloses a biodegradable barrier film comprising a non-biodegradable and water-insoluble film-forming material having a biodegradable material homogeneously dispersed throughout its structure. Water-insoluble polyvinyl alcohol and polyethylene are used for the film forming materials, while starch, dextrin, and collagen are used for the biodegradable components.

U.S. Patent 4,964,857 discloses biodegradable diapers based on multiple layers of moisture resistant paper interspersed with layers of absorbent materials. The moisture resistant paper is specified as one coated with a naturally occurring wax such as beeswax.

### SUMMARY OF THE INVENTION

The present invention encompasses flexible biodegradable films comprising composite structures derived from blends of biodegradable polymers. The biodegradable polymers are selected from the categories of moisture sensitive polymers, thermally sensitive polymers, mechanically limited polymers, polymers difficult to process into films, hydrolytically cleavable polyesters, high melting aliphatic polyesters, and degradable elastomers. The films of the present invention comprise two or more components selected from the above-mentioned categories. The biodegradable polymers are combined in various ways to overcome the deficiencies of the individual components, yet at the same time to impart specific performance properties to the film. The films are suitable for use in disposable absorbent products including diapers, adult incontinent pads, sanitary napkins, and pantiliners.

As will be discussed in detail hereinafter, the biodegradable liquid impervious monolayer films of the present invention have:
a) a machine direction (MD) tensile modulus from about 10,000 to about 100,000 lbs/sq.in. (6.895x10⁸ dynes/sq. cm. to 6.895x10⁹ dynes/sq. cm.),
b) a MD tear strength of at least 70 grams per 25.4 microns of thickness,
c) a cross machine direction (CD) tear strength of at least 70 grams per 25.4 microns of thickness,
d) an impact strength of at least 12 cm as measured by falling ball drop,
e) a moisture transport rate less than about 0.0012 grams per square centimeter per 16 hours,
f) a modulus at 60°C of at least 5.52x10⁷ dynes/sq. cm. (800 lbs/sq. in.), and
g) a thickness from about 12 microns to about 75 microns.

The present invention also encompasses disposable absorbent articles comprising a liquid pervious topsheet, a liquid impervious backsheet, and an absorbent core encased between the topsheet and the backsheet, said articles being characterized in that said backsheet comprises a flexible biodegradable film derived from blends of biodegradable polymers as will be described hereinafter.

### DETAILED DESCRIPTION OF THE INVENTION

The films used to prepare the backsheet materials and other biodegradable products employed herein are derived from combinations of two or more biodegradable polymers. In general, the biodegradable polymers alone do not meet all the performance standards required of backsheet materials. The films of the current invention may comprise blends of various biodegradable polymers, selected and compounded such that the deficiencies of the individual components are overcome. The blends are thermoplastic and can be extruded into biodegradable water resistant films suitable for backsheet applications.

The individual polymers selected for the films of the current invention are biodegradable polymers obtained from many sources, both natural and synthetic. Each polymer has certain attributes which render it biodegradable. However, many of these attributes prevent the polymer from being used singularly as a backsheet material.

For example, some biodegradable polymers are very water sensitive and lose significant strength or even dissolve when exposed to aqueous media. Examples include interpenetrated networks of destructurized starch, polyvinylalcohol and related derivatives, hydroxypropylcellulose derivatives, and polyethylene oxide.

Other biodegradable components are thermally sensitive due to their low melting points or glass transition temperatures, generally 65°C or lower. The Vicat softening temperatures of such materials can occur at temperatures much lower than 65°C, often below 45°C. Examples include many aliphatic polyesters such as polycaprolactone, polyethylene adipate, polybutylene glutarate, and polypropylene succinate.

Still other polymers have mechanical deficiencies and may be too stiff, too soft, or suffer from poor tensile and/or tear strengths. The elongational properties of some polymers are also insufficient to meet backsheet performance standards. Examples include cellulosic materials such as cellophane, cellulose esters, and blends of cellulose esters with aliphatic polyesters.

Other polymers are difficult to process by conventional means into films suitable for backsheet applications. In some cases, the crystallization rates of the polymer are too slow or the flow properties of the polymer make film processing difficult. Examples include polyhydroxy alkanoates like polyhydroxybutyrate or polyhydroxybutyrate/valerate copolymers.

Some biodegradable polymers meet many or all the physical property requirements of backsheet applications alone but do not degrade fast enough to break up into small fragments in the early stages of composting. Hence, there is a strong likelihood such polymers would be screened out of the compost stream and not become part of the final compost. Examples of such components include hydrolytically cleavable polyesters such as certain aromatic/aliphatic polyester copolymers, oxidized ethylene/carbon monoxide copolymers, and aliphatic polyesters with melting points above 65°C.

The biodegradable polymers used to fabricate the films of the current invention may also be combined with optional ingredients such as biodegradable elastomers, tie layers, pigments, processing aids and the like. Each of these components is described in detail below.

### 1. MOISTURE SENSITIVE POLYMERS

### A. DESTRUCTURIZED STARCH COMPOSITIONS

Thermoplastic, biodegradable compositions based on interpenetrated networks of starch with a synthetic component such as an ethylene/vinyl alcohol (EVOH) copolymer are described in International Patent Applications WO 90/10671, WO 90/110069.3, WO 91/02025, WO 91/02024, WO 91/02023, European Patent Application 90810533.1, and U.S. Patent 5,095,054 all herein incorporated by reference. Such materials are available commercially from Novamont under the tradename Mater-Bi and from Warner Lambert under the tradename Novon. These materials comprise greater than 50% starch by weight and are, thus, very sensitive to moisture vapor levels in the ambient atmosphere as well as direct contact with liquid water.

Films comprising only the interpenetrated network of starch and a synthetic component can be extruded with very good mechanical properties initially. However, these properties vary considerably with humidity. For example, the modulus of a Mater-Bi film (Type AF05H) decreases by about 50% as the relative humidity changes from about 20% to 90%. Although such sensitivity to humidity is a reversible process, it makes the film inconsistent on a day-to-day basis to the degree that converting operations and end-user performance are negatively affected.

Mater-Bi films also absorb water to a high degree, typically about 30% of their initial weight. In addition to lowering the strength of the film significantly, the high water absorption also leads to very high moisture transmission through the film, for example about 0.0024 grams/sq. cm/16 hours through a 30 micron film. This is beneficial in some applications where breathability is desired. However, high moisture transmission may not be desirable if the film is expected to contain large quantities of fluids, as in the case of a diaper backsheet. High water permeation can lead to excessive condensation on the outside of the backsheet leaving it cold and wet feeling to the touch.

Novon films can also be extruded with good initial properties. Some, however, like Novon grade M0014 are so sensitive to water they quickly fall apart, disperse into smaller particles, and virtually dissolve when contacted or immersed in liquid water.

### B. POLYVINYLALCOHOL (PVA) AND DERIVATIVES

Chemically, PVA can be described as a polyhydric alcohol with hydroxyl groups extending from alternate carbon atoms. It is represented structurally as follows:

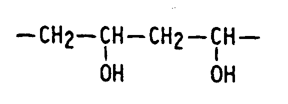

PVA is prepared via hydrolysis of polyvinylacetate. Depending on the degree of hydrolysis, PVA can be obtained in grades which are soluble in both cold and hot water or hot water only.

Unmodified PVA is not thermoplastic. However, when PVA is plasticized with appropriate additives, thermoplastic materials can be obtained. External plasticizers such as glycerol, ethylene glycol, and some of the lower polyethylene glycols are effective plasticizers for PVA.

The biodegradability of PVA is well documented. A brief overview on its biodegradation can be found in "Handbook of Water-Soluble Gums and Resins", R. L. Davidson, Editor. Chapter 20, p20-17, herein incorporated by reference.

Thermoplastic PVA compositions suitable as components for the films of the present invention are sold by Air Products and Chemicals, Inc. of Allentown, Pennsylvania, under the tradename Vinex. Vinex resins are internally plasticized compositions achieved by copolymerizing PVA with a poly(alkyleneoxy) acrylate. More detailed disclosures of these materials are given in U.S. Patents 4,618,648 and 4,675,360 both herein incorporated by reference. Still another method for making thermoplastic polyvinylalcohol compositions via the incorporation of polyurethanes is disclosed in U.S. Patent 5,028,648, herein incorporated by reference. The biodegradation of Vinex compositions is disclosed in an Air Products Technical Bulletin entitled "Measurement of the Biodegradability of Vinex Resins by Controlled Respirometry" by J. Kramer, herein incorporated by reference.

A Vinex 1000 series, 2000 series, and 3000 series exist. The 1000 series are fully hydrolyzed grades which are insoluble in cold water, but soluble in hot water. The 2000 and 3000 series are soluble in both hot and cold water. All three series of Vinex resins can be employed in films of the present invention. Especially preferred are the 2000 series. Such materials, for example Vinex 2034, form tough tear resistant films which, if not for their water solubility, would meet the mechanical strength requirements for biodegradable backsheets of absorbent articles.

In the films of the current invention, the Vinex materials are used in blends with one or more moisture resistant biodegradable polymers selected from the group consisting of thermally sensitive polymers, mechanically limited polymers, polymers difficult to process into films, hydrolytically cleavable aromatic/aliphatic polyester copolymers, oxidized ethylene/carbon monoxide copolymers, high melting aliphatic polyesters, and elastomers. The films may comprise from about 1 to about 40 weight percent of the thermoplastic polyvinyl alcohol composition.

### C. HYDROXYPROPYLCELLULOSE (HPC)

HPC is a non-ionic cellulose ether with an unusual combination of properties among cellulose derivatives. These include solubility in both water and polar organic solvents as well as plastic flow properties that permit its use for molded and extruded articles such as films. As described in aforementioned "Handbook of Water Soluble Gums and Resins" (Chapter 13), herein incorporated by reference, the plastic flow properties of HPC enable it to be used as a base material in extrusion, blow, or injection molding, and film-making operations. Thermally processed products formed by these methods retain their water solubilities, are biodegradable, and can even be made to be edible.

The chemical cellulose used to prepare HPC is derived from wood pulp or cotton linters. The cellulose is treated with aqueous sodium hydroxide to form alkali cellulose which, in turn, is reacted with propylene oxide to yield the following empirical structure: ⁅C₆H₁₀₋ₙO₅(CH₂CHOCH₃)ₙ⁆ₓ where n has a range of 3 to 4.5 and x has a range of 150 to 3000. Commercially, HPC is available from Hercules Inc. under the tradename KLUCEL.

Due to their water solubility, high stiffness, and low tear strength, HPC films alone are not acceptable for use as backsheet films for absorbent articles. HPC can be employed in blends at low levels, typically 33 weight percent or less, where it imparts temperature resistance and resistance to blocking. Small amounts of plasticizers, internal lubricants, and antioxidants can be used to improve the melt flow, mold release, and thermal stability of the polymer during processing. Suitable plasticizers include propylene glycol, glycerin, low molecular weight polyethylene glycols, and glycerol monostearate.

### II. THERMALLY SENSITIVE POLYMERS

Since commercial composting operations typically achieve temperatures of about 60°C, biodegradable polymers with low thermal softening points or melting points may be included in the films of the current invention to help break the films down into smaller fragments early in the process. Moreover, such components may also biodegrade faster than higher melting components since they are essentially liquids at composting temperatures rather than solids. The rapid initial biodegradation and loss of these components from the film would also be expected to expose more of the higher melting components to microorganisms thereby increasing their rate or biodegradation as well.

Aliphatic polyesters belong to the family of linear saturated polyesters. Many aliphatic polyesters are known to be biodegradable and, hence, compostable. Many also have melting points of about 65°C or less. Although some types of low melting aliphatic polyesters can be processed directly into thin water resistant films, their melting points are too low to allow their use alone in many applications, for example, as backsheets for disposable absorbent articles.

Polycaprolactone is an example of a preferred biodegradable aliphatic polyester for use in the present invention. It is produced via the ring opening polymerization of epsilon-caprolactone, a seven-membered ring compound. As described in Union Carbide Brochure F-60456 entitled "Tone Polymers," herein incorporated by reference, the polymerization is initiated with a diol (HO-R-OH, where R is an aliphatic segment) to produce polymers with the following structure: 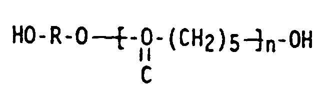 where n is the degree of polymerization.

Polycaprolactone polymers are available from Union Carbide Corporation under the tradename TONE in a variety of molecular weight grades. For example, TONE polymers P-300 and P-700 have degrees of polymerization of about 95 and 400 respectively, corresponding to molecular weights of about 10,000 and 40,000. TONE P-767 is prepared from a special high purity grade of caprolactone monomer and has an average molecular weight of about 43,000. TONE P-787 has an even higher average molecular weight of about 80,000.

Polycaprolactone polymers having molecular weights of about 40,000 and greater can be melt processed into strong water resistant films. Except for their low melting point of about 60°C (140°F), these films could function as backsheets for absorbent articles. Because of their low melting points, backsheets consisting of 100% polycaprolactone would have difficulty withstanding the high temperatures encountered when hot glue is applied to the diaper during the manufacturing process. In addition, during shipping and/or warehouse storage, temperatures of 60°C can be reached. Backsheets consisting of 100% polycaprolactone would be difficult to stabilize in such an environment and might distort, stick to one another, or even melt.

In the films of the present invention, polycaprolactone can be utilized in monolayer film blends with one or more biodegradable polymers where it may help compatibilize dissimilar materials as well as impart strength and good processability. The other biodegradable polymers can be selected from the categories of mechanically limited polymers, hydrolytically cleavable aromatic/aliphatic polyester copolymers, oxidized ethylene/carbon monoxide copolymers, and high-melting aliphatic polyesters.

In the films of the present invention, polycaprolactone polymers having an average molecular weight of 40,000 or more are preferred. Especially preferred are polycaprolactone polymers having an average molecular weight of about 80,000 grams per mole (e.g., TONE P-787).

Other types of aliphatic polyesters suitable for use in the present invention are derived from the reaction of an aliphatic dicarboxylic acid and a diol. As described in "An Overview of Plastics Degradability," by Klemchuk, published in Modern Plastics, (August, 1989) and incorporated herein by reference, many of these polyesters are biodegradable since they are susceptible to enzymatic hydrolysis. Moreover, the acid and alcohol fragments of the hydrolysis are also easily assimilated by microorganisms.

Such polyesters are prepared via the generalized reaction shown below: 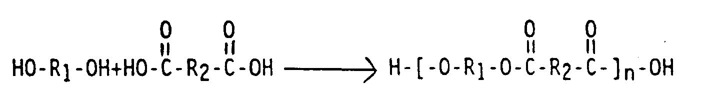 where R₁ is a linear methylene chain -(CH2-)ₓ with 2 < x < 10, R₂ is also a linear methylene chain -(CH2 -)_{y} with 2 < y < 10; and n is the degree of polymerization. Examples of these types of aliphatic polyesters include:

Polyethylene adipate where x=2 and y=4; Tm=50°C
Poly (1,3 propanediol adipate) where x=3 and y=4; Tm=38°C
Poly (1,4 butanediol adipate) where x=4 and y=4; Tm = 48°C
Poly (1,4 butanediol sebacate) where x=4 and y=8; Tm=64°C
Poly 1,3 propanediol succinate) where x=3 and y=2; Tm=47°C
Poly (1,4 butanediol glutarate) where x=4 and y=3; Tm=47°C

Further examples of thermally sensitive aliphatic polyesters with melting points less than 65°C can be found in "Polymer Handbook, Third Edition" by J. Brandrup and E.H. Immergut published by John Wiley & Sons, in Section VI, pages 56 through 67, herein incorporated by reference.

In the films of the present invention, thermally sensitive polymers are blended with one or more biodegradable polymers selected from the categories of moisture sensitive polymers, mechanically limited polymers, polymers difficult to process into films, hydrolytically cleavable aromatic/aliphatic polyester copolymers, oxidized theylene/carbon monoxide copolymers, high melting aliphatic polyesters, and elastomers. In such blends, the thermally sensitive polymer comprises from about 1 to about 60 weight percent of the blend.

### III. POLYMERS DIFFICULT TO PROCESS INTO FILMS

Another family of biodegradable aliphatic polyesters includes those derived from alpha-hydroxy carboxylic acids. This family of poly (alpha-hydroxy alkanoates) includes synthetic polymers such as polylactates from lactic acid and naturally derived polymers such as polyhydroxybutyrate (PHB) polymers and polyhydroxybutyrate/valerate (PHBV) copolymers. Preferred examples of polyhydroxybutyrate homopolymer and polyhydroxybutyrate/valerate copolymers are described in U.S. patent 4,393,167, Holmes et. al., issued July 12, 1983 and U.S. Patent 4,880,592, Martini et al., issued November 14, 1982, both references incorporated herein by reference. PHBV copolymers have the generalized structure shown below.

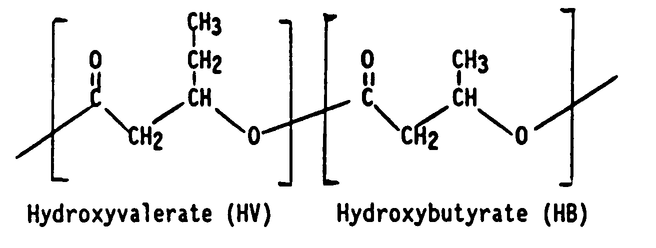

Such copolymers are commercially available from Imperial Chemical Industries under the tradename Biopol. The Biopol polymers are produced from the fermentation of sugar by the bacterium Alcaligenes eutrophus. PHBV polymers are currently produced with valerate contents ranging from about 5 to about 24 mole percent. Increasing valerate content decreases the melting point, crystallinity, and stiffness of the polymer. An overview of Biopol technology is provided in Business 2000+, (Winter, 1990), incorporated herein by reference.

Unfortunately PHBV copolymers are difficult to process directly into films because of their slow crystallization rate. This causes the film to stick to itself even after cooling to room temperature because a substantial fraction of the PHBV remains amorphous and tacky for long periods of time. In cast film operations, where the film is immediately cooled on chill rolls after leaving the film die, molten PHBV often sticks to the rolls restricting the speed at which the film can be processed, or even preventing the film from being collected. In blown films, residual tack of the PHBV causes the tubular film to stick to itself after it has been cooled and collapsed for winding.

International Patent Application 86309586.5 describes a means of achieving a PHBV monolayer film for diaper backsheet applications by coextruding the PHBV between two layers of sacrificial polymer, for example a polyolefin, stretching and orienting the multilayer film, and then stripping away the polyolefin layers after the PHBV has had time to crystallize. The remaining PHBV film is then laminated to either water soluble films (PVA preferred) or water insoluble films such as polyvinylidene chloride or other polyolefins.

In the films of the current invention, such drastic processing methods are avoided. In monolayer films, the PHBV component is generally employed in an amount less than about 40% by weight with one or more polymers selected from the category of polymers more readily processed into films consisting of moisture sensitive polymers, thermally sensitive polymers, mechanically limited polymers, hydrolytically cleavable aromatic/aliphatic polyester copolymers, oxidized ethylene/carbon monoxide copolymers, high melting aliphatic polyesters, and elastomers. This allows the other components to function as the continuous phase in the blend and overcome the tackiness and processing difficulties of the PHBV as described above.

### IV. MECHANICALLY LIMITED POLYMERS

### A. CELLULOSE ESTERS

Cellulose esters are produced by the chemical modification of cellulose and include the family of cellulose acetates (CA), cellulose acetate propionates (CAP), and cellulose acetate butyrates (CAB). As described in the 1990 Modern Plastics Encyclopedia (Mc Graw-Hill, pp. 23-24), herein incorporated by reference, cellulose esters are prepared by reacting cellulose with particular acids and acid anhydrides, generally in the presence of a sulfuric acid catalyst. In the case of CA, the reaction is first carried out with acetic acid and acetic anhydride to produce cellulose triacetate, which contains nearly 100% acetyl substitution or, in other words, a degree of substitution of about 3.0. The triacetate is then partially hydrolyzed to remove some of the acetyl groups such that the CA product contains about 38 to 50% acetyl substitution.

CAP and CAB are made by substituting propionic acid and propionic anhydride or butyric acid or butyric anhydride for some of the acetic acid and acetic anhydride. Plastic grades of CAP generally contain 39 to 47% propionyl and 2 to 9% acetyl content. Plastic CAB grades generally contain 26 to 39% butyryl and 12 to 15% acetyl content. Commercially, CA, CAB, and CAP are obtained from Eastman Chemical Products, Inc. under the tradename Tenite.

Although raw cellulose and its regenerated film (cellophane) and fiber (rayon) forms are readily biodegradable, the esterification of cellulose can make it quite stable to microbial attack. As described in "Polymer Degradation" by W. Schnabel (Macmillan, New York, 1981), herein incorporated by reference, this enhanced resistance to biodegradation results from the inability of cellulose-specific enzymes to attack the substituted portions of the polysaccharide. However, as described by Buchanan and Gardner in an Abstract of their paper entitled " The Fate of Cellulose Esters in the Environment: Aerobic Biodegradation of Cellulose Acetate" presented at the CELLULOSE '91 CONFERENCE held in New Orleans, Louisiana, December 2-6,1991, the rate of degradation of cellulose esters also depends upon the degree of substitution. For example, a CA with a 1.7 degree of substitution was found to biodegrade much faster than a CA with a 2.5 degree of substitution.

Fully formulated grades of cellulose esters may also contain plasticizers, heat stabilizers, and ultraviolet inhibitors. High levels of these stabilizers and inhibitors may further slow the rate of biodegradation of cellulose esters. Zero or very low levels of such stabilizers are generally preferred in biodegradable films.

Plasticized cellulose esters like CA, CAP, and CAB are thermoplastic and can be melt processed into thin films. Unless substantial levels of plasticizer are employed, the stiffness of such films is too high for them to be useful as backsheets for absorbent articles. Even in the presence of plasticizers, the tear propagation resistance of cellulose ester films is very low, typically below 20 grams per 25.4 microns of thickness in the machine direction.

In films of the current invention, cellulose esters are used in blends with one or more biodegradable polymers selected from the category of moisture sensitive polymers, thermally sensitive polymers, hydrolytically cleavable aromatic/aliphatic polyester copolymers, oxidized ethylene/carbon monoxide copolymers, and elastomers. In binary blends with elastomers, the film may comprise 30-70 weight percent of a cellulose ester and 70-30 weight percent of the elastomer.

### B. CELLULOSE ESTER BLENDS WITH OTHER BIODEGRADABLE POLYMERS

It is well known that cellulose esters form miscible blends with many aliphatic polyesters. U.S. patent 3,642,507, herein incorporated by reference, discloses the formulation of printing inks with improved flexibility by blending a cellulose ester with polycaprolactone. U.S. patent 3,922,239, herein incorporated by reference, also discloses the preparation of thermoplastic blends of cellulose esters and polycaprolactone and other cyclic ester polymers. The addition of the polyesters was found to lower the modulus of the blend significantly below that of the cellulose ester and to impart improved melt processability, toughness, and impact resistance.

More recently, blends of CAP and CAB with polyhydroxybutyrate (PHB) have been described by Ceccorulli, Pizzoli, and Scandola in an Abstract of a paper entitled "Blends of Cellulose Esters with Bacterial Poly(3-hydroxybutyrate)" presented at the aforementioned CELLULOSE '91 CONFERENCE, and herein incorporated by reference. Experimental evidence of miscibility was found up to 50% PHB. Crystallization of the PHB was found to be strongly inhibited by the presence of cellulose esters confirming intimate mixing of the blend components. Similar results are obtained if PHBV copolymers are employed in place of PHB.

Blends as described above are thermoplastic and can be processed into thin flexible films with stiffness levels appropriate for backsheet films. However, the tear propagation resistance of such films alone is still deficient compared to those normally used to construct absorbent articles such as disposable diapers. As will be discussed later, the inclusion of certain biodegradable elastomers can improve the tear strength of said blends significantly.

### V. HYDROLYTICALLY CLEAVABLE POLYESTERS

### A. Aromatic/Aliphatic Polyester Copolymers

Aromatic polyesters such as polyethylene terephthalate (PET) and polybutylene terephthalate (PBT) are leading examples of high performance polyesters for film, fiber, or injection molding applications. Although the monomeric starting materials for such polyesters are known to be biodegradable, neither PET or PBT are considered to be biodegradable polymers. As homopolymers, both polyesters tend to crystallize to relatively high extents making them extremely difficult to hydrolyze except under conditions of very high temperature in the presence of water, for example in the melt state, or in the presence of strongly acidic or basic media. Such conditions do not exist in municipal composting operations. In the case of PET, not only is a significant fraction of the polymer in a crystalline state, but the softening point or glass transition temperature (about 80°C) of the remaining noncrystalline or amorphous fraction is also above typical composting temperatures. Hence, even the amorphous fraction will be resistant to hydrolysis in a composting operation.

Several means of making aromatic polyesters more readily hydrolytically cleavable, and hence more likely to be biodegradable, have recently been described. U.S. Patent 5,053,482 issued to Tietz on October 1, 1991 describes polyesters based on polyethylene terephthalate (PET) copolymerized with diethylene glycol and 5-sulfoisophthalic acid wherein the the glass transition temperature of the copolymers is preferrably reduced to below 65°C, within the range of normal composting operations. The copolymers are said to be suitable for producing films and fibers for use in disposable articles such as diapers. Although hydrolysis of the copolymer films and fibers is shown to take place in boiling water, (100°C) evidence of the extent or rate to which true biodegradation occurs is not presented.

Yet another approach to increasing the hydrodegradability of aromatic polyesters is described in International Patent Application WO 91/02015 published February 21, 1991, herein incorporated by reference. In this case, hydrodegradable polyesters based on the random copolymerization of aromatic and aliphatic polyesters is disclosed. More specifically, the random copolymers are comprised of aromatic polyesters such as PET or PBT randomly interrupted with aliphatic hydrodegradable link polymers such as polyglycolic acid, polylactic acid, polycaprolactone, polyhydroxybutyrate, polyhydroxybutyratevalerate, polybutylene oxalate, polyethylene adipate, polyethylene carbonate, polybutylene carbonate, and other polyesters containing silyl ethers, acetals, or ketals. Preparation of the copolymers is carried out by either ester interchange reactions of the appropriate monomeric species or by transesterification reactions beween two homopolymers in the presence of an appropriate catalyst.

In addition to the aforementioned aliphatic link polymers, other aliphatic polyesters may also be appropriate for producing aromatic/aliphatic polyester copolymers. These include aliphatic polyesters selected from the group of oxalates, malonates, succinates, glutarates, adipates, pimelates, suberates, azelates, sebacates, nonanedioates, and mixtures thereof.

In both the above cases, it is assumed true biodegradation will occur once the copolymers hydrolyze to very low molecular weight oligomers or monomeric species. However, the rate to which this occurs in a composting environment at temperatures below 65°C and at relatively small deviations from neutral pH may be too slow to ensure the breakup of films before they are removed by screening, air classification, or other separations method. To enhance the initial breakup and ultimate degradation of such polyester copolymers, blending with other more rapidly biodegradable polymers can be employed, said more rapidly biodegradable polymers selected from the categories of moisture sensitive polymers, thermally sensitive polymers, polymers difficult to process into films, and mixtures thereof. In such blends, the aromatic/aliphatic polyester copolymer comprises from about 60 weight percent to about 95 weight percent of the blend.

### B. OXIDIZED ETHYLENE-CARBON MONOXIDE COPOLYMERS

Photodegradable polymers based on random copolymers of ethylene and carbon monoxide are used commercially in beverage can rings and other items which may often be discarded in the environment as litter. Such copolymers are disclosed in U.S. Patent 2,495,286 issued to Brubaker, herein incorporated by reference. In order to provide acceptable rates of photodegradation, only a small proportion (generally 1 to 3 mole percent) of carbon monoxide or other carbonyl functionality needs to be incorporated into the polymer. Decomposition of such plastics begins as soon as the plastic is exposed to sunlight although there may be a delay period before significant breakdown of important physical properties are noted. As the polymer chains decrease in molecular weight, the plastic material macroscopically breaks down into smaller fragments which become more and more susceptible to microbial attack and hence biodegradation.

In the absence of sunlight, for example, in a commercial composting operation, this photodegradative process will not be initiated and the plastic articles containing photodegradable linkages will not break down in both molecular weight and size rapidly to become part of the final compost. Rather, they will likely be screened out or otherwise separated from the compost stream along with other non-biodegradable materials.

Recently, it has been found that ethylene/carbon monoxide polymers can be oxidized to yield a new type of aliphatic polyester. U.S. Patent 4,929,711, herein incorporated by reference, describes a process for converting a polyketone, for example a ethylene/carbon monoxide (ECO) copolymer, to a polyester. The process involves the reaction of the ECO copolymer with an organic peroxyacid oxidizing agent in an inert liquid medium at temperatures between -20°C to 150°C. Substantially all or only a portion of the ketone functionality can be converted to ester groups depending upon the reaction conditions.

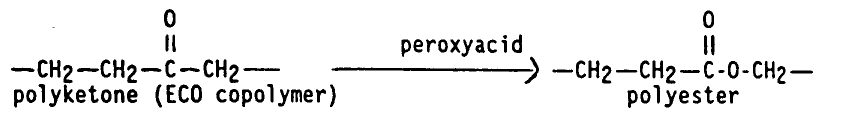

Subsequent U.S. Patent 4,957,997 expands this process to polyketones containing pendant functional groups obtained by the copolymerization of carbon monoxide with vinyl or vinylidene monomers. The vinyl or vinylidene monomer may have at least one functional group containing one or more oxygen, nitrogen, sulfur, or halogen atoms.

The new polyesters described in these patents may originate from polyketones containing 0.5 to 20 weight percent carbon dioxide and having a molecular weight from about 10,000 to about 1,000,000 grams per mole. After oxidation to the corresponding polyesters, the materials are capable of being hydrolyzed to lower molecular weight fragments. The rate and extent to which both hydrolytic and microbial degradation occur depend on the number of ester groups present and the average molecular weight of the polymer between ester groups. The lower the molecular weight fragments resulting from hydrolysis become, the more susceptible they become to microbial attack and biodegradation. Preferably, the average molecular weight of the polymer chains between ester groups is below 1000 grams per mole. Most preferably, the average molecular weight between ester groups is below about 500 grams per mole.

Oxidized ECO copolymers have excellent moisture resistance and processability but their physical properties, particularly elongation to break in tension, at lower molecular weights, may not be sufficient to be used alone for backsheets of absorbent articles. As mentioned in previous sections of this application, oxidized ECO copolymers are useful as blend components with other biodegradable polymers where they impart heat resistance and moisture resistance. Oxidized ECO copolymers can be employed in amounts ranging from 1 to 99 weight percent of the total blend composition.

### C. High Melting Aliphatic Polyesters

Yet another family of hydrolytically cleavable and biodegradable polyesters are high melting aliphatic polyesters defined, herein, as those having glass transition temperatures or melting points above 65°C. Such materials may not undergo initial decomposition and breakup during the early stages of composting since the crystalline, fraction of these materials, or the amorphous fraction, or both the crystalline and amorphous fractions may be below their melting points or glass transitions at normal composting temperatures. High melting aliphatic polyesters can, however, be combined in blends with other more rapidly degrading materials, for example moisture sensitive or thermally sensitive polymers, to enhance their rates of initial decomposition and breakup. Examples of high melting aliphatic polyesters include polyethylene sebacate (Tm=76°C), polyethylene succinate (Tm= 108°C), and polyhexamethylene sebacate (Tm = 78°C). Further examples can be found in the aforementioned "Polymer Handbook - Third Edition" Section VI, pages 56 through 67, previously incorporated herein by reference. High melting aliphatic polyesters can be used in blends with other biodegradable polymers wherein the high melting aliphatic polyester comprises from about 1 to about 99 weight percent of the blend.

### VI. ELASTOMERS

In the case of the mechanically deficient materials (cellulose esters and cellulose ester blends with various aliphatic polyesters) discussed earlier, it was noted their main deficiency was in tear strength. One way of improving the tear strength of films made from such materials is to incorporate a suitable thermoplastic elastomer (TPE) into the material by, for example, melt blending. A thermoplastic elastomer is a material that combines the processability of a thermoplastic with the functional performance and properties of a conventional thermosetting elastomer as discussed in Modern Plastics Encyclopedia, 1990, pp 122-131, herein incorporated by reference. Commercially, there are 6 generic classes of TPE: styrenic block copolymers, polyolefin blends, elastomeric alloys, thermoplastic polyurethanes, thermoplastic copolyesters, and thermoplastic polyamides.

For use in films of the present invention, any thermoplastic elastomer incorporated into the film must be biodegradable. From the aforementioned list of TPE classes, only a select group of thermoplastic polyurethanes, specifically aliphatic polyester-based polyurethanes, are generally recognized as being biodegradable.

Biodegradable polyurethanes can be prepared from low molecular weight aliphatic polyesters derived from epsilon-caprolactone or the reaction products of a diol-dicarboxylic acid condensation. In general, these polyesters have molecular weights less than 10,000 grams per mole and frequently as low as 1000 to 2000 grams per mole. Examples of biodegradable polyester urethanes derived from polyethyleneglycol adipate, poly (1,3-propanediol adipate) and poly (1,4-butanediol adipate) are disclosed in "The Prospects for Biodegradable Plastics" by F. Rodriguez (Chem Tech, July - 1971) incorporated herein by reference.

Aliphatic polyester urethanes are available from Morton International, Inc. under the tradename Morthane. When blended with other biodegradable polymers such as moisture sensitive polymers, thermally sensitive polymers, mechanically limited polymers, hydrolytically cleavable aromatic/aliphatic polyester copolymers, oxidized ethylene/carbon monoxide copolymers, high melting aliphatic polyesters, and mixtures thereof, they lower modulus, increase tear and impact strength, and impart moisture resistance. The aliphatic polyester urethanes may comprise 20-80 weight percent of the blends.

Procedures for synthesizing another type of TPE suitable for imparting improved toughness and tear strength to cellulose ester and cellulose ester blend films are disclosed in U.S. Patent 3,585,257 issued to Mueller et al and herein incorporated by reference. Block copolymers of polycaprolactone with polydienes such as polyisoprene and polybutadiene are disclosed in which the polycaprolactone content can be varied from about 20 to about 80 weight percent and the diene content varied from about 80 to about 20 weight percent. Copolymers having tensile strengths in the range between 245 and 2000 pounds per square inch and elongations to break in the range from 400 to 560 percent are obtained.

Block copolymers can be prepared having various architectures. For example an A-B diblock copolymer comprises a block of polymer A segments coupled to a block of B polymer segments. An A-B-A triblock copolymer comprises a block of B segments coupled to a block of A segments at each of its terminal ends. An --(A-B)ₙ -- multiblock copolymer comprises alternating sequences of A and B segments where n=2,3, etc.

For toughening and increasing the tear strengths of films of the present invention, A-B-A triblock or --(A-B)ₙ -- multiblock copolymers in which polycaprolactone segments comprise the A blocks and n = 2, 3, etc. are generally preferred. Simple diblock A-B copolymers do not impart significant tear strength improvement to films of the present invention. Especially preferred are triblock copolymers in which the polycaprolactone segments comprise from about 10 to about 60 weight percent of the copolymer and the polydiene segments comprise from about 90 to about 40 weight percent of the copolymer. To achieve the desired tear strength enhancement the copolymers are melt compounded with a cellulose ester or cellulose ester blend such that the copolymer comprises from about 30 to about 70 weight percent of the total composition.

### VIII. OPTIONAL COMPONENTS

In addition to the above-mentioned components, the backsheet films of the present invention may contain other components as may be, or later become, known in the art, including, but not limited to, antiblocking agents, antistatic agents, slip agents, pro-heat stabilizers, antioxidants, pro-oxidant additives, pigments, plasticizers, etc.

Antiblocking agents act to prevent film layers from sticking to one another when wound into a roll or when disposable articles are packaged in contact with one another. Typical antiblocking substances include concentrates of silica or talc blended with a polymeric material such as polyethylene or polycaprolactone. Reduction of blocking in the films of the present invention can also be obtained by loading the film surface with small particles or powders such as chalk, clay, silica, starch, and similar materials. Powdered polymeric materials (e.g., polytetrafluoroethylene) can also be used to reduce blocking when applied to the surface of films of the present invention. Such film surface treatments can be used to reduce blocking alone or in combination with other antiblock methods. The quantity of powder antiblock substance commonly added to the surface of a film, when used, is from about 0.5 g/m2 to about 5 g/m2.

Antistatic agents may be incorporated in films of the present invention; examples of such agents include ethoxylated amines and quarternary amine salts having organic constituents of about 12-18 carbon atoms in length. Agents of this type slowly defuse to the surface of the film and, because of their ionic character, form an electrically conductive layer on the surface of the film. Antistatic agents commonly constitute from about 1% to about 5% of the weight of the films, when used.

Slip agents may be incorporated into the films of the present invention to reduce drag over rollers and other forming equipment. Examples of such agents are those commonly derived from amides of fatty acids having about 12 to 22 carbon atoms. Such agents may augment the antiblocking properties of the films of the present invention. Such slip agents are commonly incorporated in films from about 0.05% to about 3% of the weight of the films when used.

### PERFORMANCE CRITERIA AND TEST METHODS

For a film to perform satisfactorily as a compostable disposable diaper backsheet, it must be made of resins or structures that are biodegradable and it must demonstrate the following properties of high strength, adequate fluid barrier, appropriate modulus or flexibility, and adequately high melting point.

The backsheets of disposable diapers must have sufficient strength both to process on a high speed disposable diaper converting machine and to provide a "wetproof" barrier in use on an infant. It must be sufficiently wetproof so that clothing or bedding, either that of the infant or of the caregiver, is not wet or soiled. It must have a modulus or flexibility that is, at the same time, low enough to be a soft, pleasing material to be used as the outer covering of an infant diaper yet high enough to handle easily on high speed disposable diaper converters without wrinkling, folding, or creasing. It must have sufficient resistance to heat such that it will not deform, melt, or permanently loose strength in typical hot storage conditions or loose its integrity on high speed disposable diaper converters which typically use hot melt adhesives to bond the components of a disposable diaper together.

It has been found that films that are sufficiently strong to be suitable as biodegradable backsheets for disposable diapers demonstrate two properties: (a) resistance to rupture from a dropped weight and (b) resistance to tearing in both the machine direction of manufacture and the across-machine direction of manufacture. Acceptable compostable backsheets can withstand the drop of a spherical steel ball of about 19 millimeters in diameter and 27.6 to 28.6 gram mass from a height of 12 centimeters so that at least 50% of the tests result in no rupture of any size (deformation is acceptable). Preferred materials are those that exhibit 50% or less failures from a height of more than 20 centimeters. Similarly, acceptable biodegradable backsheets demonstrate an average tear propogation resistance of 70 grams per 25.4 micron thickness of material in both the machine direction and cross-machine direction of manufacture when a standard Elmendorf pendulum-type test device, such as Elmendorf Model No. 60-100, is employed against 16 plies of material which has been prepared with a cut or notch according to TAPPI Method T 414om-88. More preferable, are those backsheets that demonstrate tear propogation resistances of 200 or more grams per 25.4 micron thickness in the cross-machine direction because these are particularly good at avoiding a tendency to fail in use by splitting.

It has also been found that films of sufficient barrier to moisture transport are those that permit less than 0.0012 grams of synthetic urine to pass into an absorbent paper towel per square centimeter of area per 25.4 micron thickness for every 16 hours of time when the test film is located between the absorbent paper towel and a typical absorbent gelling material-containing diaper core and a pressure simulating that of a baby. The specific conditions of the test are that the area of the core is larger than that of the test material, the core is loaded with synthetic urine to its theoretical capacity and it is under a weight of about 35 g/sq cm (0.5 psi).

It has also been found that materials of sufficient heat resistance demonstrate a Vicat softening point of at least 45°C. Vicat softening is tested using a Heat Distortion Apparatus Model No. CS-107 or equivalent and a modification of ASTM D-1525. The modification is in the preparation of the sample. A 19 millimeter by 19 millimeter size film of 4.5 to 6.5 micron thickness is prepared for Vicat needle penetration tests by melting the material to be tested into a mold of using a temperature of 120°C. and pressure of 7.031x10⁵ g/sq cm (10,000 psi) (using a Carver or similar press) for two minutes after a warmup period of at least 2 minutes. The Vicat softening point is the temperature at which a flat-ended needle of 1 sq mm circular cross section will penetrate the sample to a depth of 0.1 cm under a load 1000 g using a uniform temperature rise rate of 50°C per hour.

It has also been found that materials of sufficient machine direction modulus demonstrate a 1% secant-type modulus above at least about 6.895x10⁸ dynes/sq cm (10,000 psi) and below about 6.895x10⁹ dynes/sq cm (100,000 psi). The test is performed on an electronic tensile test machine such as the Instron Model 4201. A 2.54 cm wide strip of material, preferably of 0.00254 cm in thickiness, is cut to a length of about 30 cm with the longer dimension parallel to the machine direction of the material. The test strip is clamped into the jaws of the tensile testor so that the gauge or actual length of the material tested is 25.4 cm. The jaws are separated at a slow speed in the range of 2.54 cm per minute to 25.4 cm per minute and a stress-strain curve is plotted on a chart within an attached recording device. The 1% secant modulus is determined by reading the stress or tensile from the chart at the 1% elongation strain point. for example, the 1% strain point is achieved when the distance between jaws has increased by 0.254 cm. When the jaws are separating at the rate of 2.54 cm per minute and the recording device is running at a speed of 25.4 cm per minute, the 1% strain point will be located at a distance of 2.54 cm from the initial point. The tensile response is divided by the thickness of the sample material if it is not 0.00254 cm in thickness. Particularly soft, and therefore preferred, materials exhibit 1% secant moduli in the range of 6.895x10⁸ to 2.068x10⁹ dynes/sq cm (10,000 to 30,000 psi).

Since absorbent articles may experience temperatures as high as 140°F (60°C) during warehouse storage or shipping in trucks or railcars, it is important that the backsheet film and other components be retain their integrity at these temperatures. Although it is expected that the modulus of the films will decrease somewhat between 20°C and 60°C, the modulus should not decrease too far and allow the film to deform in the package before it reaches the end user.

For example, a polyethylene backsheet with a room temperature modulus of about 4x10⁹ dynes/sq. cm. (58,000 psi) may have a 60°C modulus of 1.2x10⁹ dynes/sq. cm. (18,560 psi) which is acceptable. A softer polyethylene backsheet with a room temperature modulus of about 8.0x10⁸ dynes/sq.cm.(11,600 psi) may have a 60°C modulus of about 3.5x10⁸ dynes/sq. cm. (5076 psi) which is still acceptable. In general, an acceptable backsheet film will have a 60°C modulus of at least 5.52x10⁸ dynes/sq.cm. (800 psi).

The modulus dependence on temperature, also called a modulus/temperature spectrum, is best measured on a dynamic mechanical analyzer (DMA) such as a Perkin Elmer 7 Series/Unix TMA 7 Thermomechanical Analyzer equipped with a 7 Series/Unix DMA 7 Temperature/Time software package, hereinafter referred to as the DMA 7, available from the Perkin-Elmer Corporation of Norwalk, Connecticut. Many other types of DMA devices exist, and the use of dynamic mechanical analysis to study the modulus/temperature spectra of polymers is well known to those skilled in the art of polymer characterization. This information is well summarized in two books, the first being "Dynamic Mechanical Analysis of Polymeric Material, Materials Science Monographs Volume 1" by T. Murayama (Elsevier Publishing Co., 1978) and the second being " Mechanical Properties of Polymers and Composites, Volume 1" by L.E. Nielsen (Marcel Dekker, 1974), both incorporated herein by reference.

The mechanism of operation and procedures for using the DMA 7 are found in Perkin-Elmer Users' Manuals 0993-8677 and 0993-8679, both dated May, 1991, and both herein incorporated by reference. To those skilled in the use of the DMA 7, the following run conditions should be sufficient to replicate the 60°C modulus data presented hereinafter.

To measure the modulus/temperature spectrum of a film specimen, the DMA 7 is set to run in temperature scan mode and equipped with an extension measuring system (EMS). A film specimen approximately 3 mm wide, 0.0254 mm thick, and of sufficient length to allow 6 to 8 mm of length between the specimen grips is mounted in the EMS. The apparatus is then enclosed in an environmental chamber swept continuously with helium gas. Stress is applied to the film in the length direction to achieve a deformation or strain of 0.1 percent of the original length. A dynamic sinusoidal strain is applied to the specimen at a frequency of 5 cycles per second. In the temperature scan mode, the temperature is increased at a rate of 3.0°C/minute from 25°C to the point where the specimen melts or breaks, while the frequency and stress are held constant. Temperature-dependent behavior is characterized by monitoring changes in strain and the phase difference in time between stress and strain. Storage modulus values in Pascals (1 Pascal= 10 dynes/sq. cm.) are calculated by the computer along with other data and displayed as functions of temperature on a video display terminal. Normally the data are saved on computer disk and a hard copy of the storage modulus/temperature spectrum printed for further review. The 60°C modulus is determined directly from the spectrum.

### METHOD OF FILM MANUFACTURE

The films of the present invention used as biodegradable backsheets may be processed using conventional procedures for producing films of blended polymers on conventional film making equipment. Pellets of the above described components can be first dry blended and then melt mixed in the film extruder itself. Alternatively, if insufficient mixing occurs in the film extruder, the pellets can be first dry blended and then melt mixed in a precompounding extruder followed by repelletization prior to film extrusion.

The polymer blends can be melt processed into films using either cast or blown film extrusion methods both of which are described in "Plastics Extrusion Technology" -- 2nd Ed., by Allan A. Griff (Van Nostrand Reinhold -- 1976), herein incorporated by reference. Cast film is extruded through a linear slot die. Generally the flat web is cooled on a large moving polished metal roll. It quickly cools, and peels off this first roll, passes over one or more auxiliary cooling rolls, then through a set of rubber-coated pull or "haul-off" rolls, and finally to a winder. A method of making a cast backsheet film for the absorbent products of the current invention is described in Example 1 which follows.

In blown film extrusion the melt is extruded upward through a thin annular die opening. This process is also referred to as tubular film extrusion. Air is introduced through the center of the die to inflate the tube and causes it to expand. A moving bubble is thus formed which is held at constant size by control of internal air pressure. The tube of film is cooled by air blown through one or more chill rings surrounding the tube. The tube is next collapsed by drawing it into a flattening frame through a pair of pull rolls and into a winder. For backsheet applications the flattened tubular film is subsequently slit open, unfolded, and further slit into widths appropriate for use in absorbent products.

Both cast film and blown film processes can be used to produce either monolayer or multilayer film structures. For the production of monolayer films from a single thermoplastic material or blend of thermoplastic components only a single extruder and single manifold die are required.

### ABSORBENT ARTICLES

Film materials used as liquid impervious backsheets in absorbent articles, such as disposable diapers, typically have a thickness of from 0.01 mm to about 0.2 mm, preferably from 0.012 mm to about 0.051 mm.

In general, the liquid impervious backsheet is combined with a liquid pervious topsheet and an absorbent core positioned between the topsheet and the backsheet. Optionally, elastic members and tape tab fasteners can be included. While the topsheet, the backsheet, the absorbent core and elastic members may be assembled in a variety of well known configurations, a preferred diaper configuration is described generally in U.S. Patent 3,860,003, entitled "Contractible Side Portion for Disposable Diaper" which issued to Kenneth B. Buell on January 14, 1975 and which patent is incorporated herein by reference.

The topsheet is compliant, soft-feeling, and non-irritating to the wearer's skin. Further, the topsheet is liquid pervious, permitting liquids to readily penetrate through its thickness. A suitable topsheet may be manufactured from a wide range of materials such as porous foams, reticulated foams, apertured plastic films, natural fibers (e.g., wood or cotton fibers), synthetic fibers (e.g., polyester or polypropylene fibers) or from a combination of natural and synthetic fibers. Preferably, the topsheet is made of a hydrophobic material to isolate the wearer's skin from liquids in the absorbent core.

A particularly preferred topsheet comprises staple-length polypropylene fibers having a denier of about 1.5 such as Hercules type 151 polypropylene marketed by Hercules, Inc. of Wilmington, Delaware. As used herein, the term "staple-length fibers" refers to those fibers having a length of at least about 16 mm.

There are a number of manufacturing techniques which may be used to manufacture the topsheet. For example, the topsheet may be woven, nonwoven, spunbonded, carded, or the like. A preferred topsheet is carded, and thermally bonded by means well known to those skilled in the fabrics art. Preferably, the topsheet has a weight from about 18 to about 25 g/m², a minimum dried tensile strength of at least about 400 g/cm in the machine direction, and a wet tensile strength of at least about 55 g/cm in the cross-machine direction.

The topsheet and the backsheet are joined together in any suitable manner. As used herein, the term "joined" encompasses configurations whereby the topsheet is directly joined to the backsheet by affixing the topsheet directly to the backsheet, and configurations whereby the topsheet is indirectly joined to the backsheet by affixing the topsheet to intermediate members which in turn are affixed to the backsheet. In a preferred embodiment, the topsheet and the backsheet are affixed directly to each other in the diaper periphery by attachment means such as an adhesive or any other attachment means as known in the art. For example, a uniform, continuous layer of adhesive, a patterned layer of adhesive, or an array of separate lines or spots of adhesive may be used to affix the topsheet to the backsheet.

Tape tab fasteners are typically applied to the back waistband region of the diaper to provide a fastening means for holding the diaper on the wearer. The tape tab fasteners can be any of those well known in the art, such as the fastening tape disclosed in U.S. Patent 3,848,594, issued to Kenneth B. Buell on November 19, 1974, the disclosure of which is incorporated herein by reference. These tape tab fasteners or other diaper fastening means are typically applied near the corners of the diaper.

Preferred diapers have elastic members despised adjacent the periphery of the diaper, preferably along each longitudinal edge so that the elastic members tend to draw and hold the diaper against the legs of the wearer. The elastic members are secured to the diaper in an contractible condition so that in a normally unrestrained configuration the elastic members effectively contract or gather the diaper. The elastic members can be secured in an contractible condition in at least two ways. For example, the elastic members may be stretched and secured while the diaper is in an uncontracted condition. Alternatively, the diaper may be contracted, for example, by pleating, an elastic member secured and connected to the diaper while the elastic members are in their relaxed or unstretched condition.

The elastic members may take a multitude of configurations. For example, the width of the elastic members may be varied from about 0.25 mm to about 25 mm or more; the elastic members may comprise a single strand of elastic material or the elastic members may be rectangular or curvilinear. Still further, the elastic members may be affixed to the diaper in any of several ways which are known in the art. For example the elastic members may be ultrasonically bonded, heat and pressure sealed into the diaper using a variety of bonding patterns, or the elastic members may simply be glued to the diaper.

The absorbent core of the diaper is positioned between the topsheet and backsheet. The absorbent core may be manufactured in a wide variety of sizes and shapes (e.g., rectangular, hour-glass, asymmetrical, etc.) and from a wide variety of materials. The total absorbent capacity of the absorbent core should, however, be compatible with the designed liquid loading for the intended use of the absorbent article or diaper. Further, the size and absorbent capacity of the absorbent core may vary to accommodate wearers ranging from infants through adults.

A preferred embodiment of the diaper has a hour-glass shaped absorbent core. The absorbent core is preferably an absorbent member comprising a web or batt of airfelt, wood pulp fibers, and a particulate absorbent polymeric composition disposed therein.

Other examples of absorbent articles according to the present invention are sanitary napkins designed to receive and contain vaginal discharges such as menses. Disposable sanitary napkins are designed to be held adjacent to the human body through the agency of a garment, such as an undergarment or a panty or by a specially designed belt. Examples of the kinds of sanitary napkins to which the present invention is readily adapted are shown in U.S. Patent 4,687,478, entitled "Shaped Sanitary Napkin With Flaps" which issued to Kees J. Van Tilburg on August 18, 1987, and in U.S. Patent 4,589,876, entitled "Sanitary Napkin" which issued to Kees J. Van Tilburg on May 20, 1986, the disclosures of both patents being incorporated herein by reference. It will be apparent that the polymeric biodegragable films described herein may be used as the liquid impervious backsheet of such sanitary napkins. On the other hand it will be understood the present invention is not limited to any specific sanitary napkin configuration or structure.

In general, sanitary napkins comprise a liquid impervious backsheet, a liquid pervious topsheet, and an absorbent core placed between the backsheet and the topsheet. The backsheet comprises one of the biodegradable films containing a blend of polymeric components as described above. The topsheet may comprise any of the topsheet materials discussed with respect to diapers.

Importantly, the absorbent articles according to the present invention are compostable to a greater extent than conventional absorbent articles which employ a polyolefin, typically a polyethylene backsheet.

The term "compostable" as used herein means a material that meets the following three requirements: (1) is capable of being processed in a composting facility for solid waste; (2) if so processed will end up in the final compost; and (3) if the compost is used in the soil the material will ultimately biodegrade in the soil.

A polymer film material present in solid waste submitted to a composting facility for processing does not necessarily end up in the final compost. Certain composting facilities subject the solid waste stream to air classification prior to further processing, in order to separate paper and other materials. A polymer film would most probably be separated from the solid waste stream in such an air classification and therefore not be processed in the composting facility. Nevertheless, it may still be a "compostable" material according to the above definition because it is "capable" of being processed in a composting facility.

The requirement that the material ends up in the final compost typically means that it undergoes a form of degradation in the composting process. Typically, the solid waste stream will be subjected to a shredding step in an early phase of the composting process. As a result, the polymer film will be present as shreds rather than a sheet. In the final phase of the composting process, the finished compost will be subjected to a screening step. Typically, the polymer shreds will not pass through the screens if they have retained the size they had immediately after the shredding step. The compostable materials of the present invention will have lost enough of their integrity during the composting process to allow semidegraded shreds to pass through the screens. However, it is conceivable that a composting facility might subject the solid waste stream to a very rigorous shredding and a rather coarse screening, in which case nondegradable polymers like polyethylene would meet requirement (2). Therefore, meeting requirement (2) is not enough for a material to be compostable within the present definition.

What does distinguish the compostable material as defined herein from materials like polyethylene is requirement (3) that they ultimately biodegrade in the soil. This biodegradation should be complete to CO₂ and water. This biodegradability requirement is not essential to the composting process or the use of composting soil. Solid waste and the compost resulting therefrom may contain all kinds of nonbiodegradable materials, for example, sand. However, to avoid a build up of man-made materials in the soil, it is required herein that such materials be fully biodegradable. By the same token, it is not at all necessary that this biodegradation be fast. As long as the material itself and intermediary decomposition products are not toxic or otherwise harmful to the soil or the crops, it is fully acceptable that their biodegradation takes several months or even years, since this requirement is present only to avoid an accumulation of man-made materials in the soil.

The following examples illustrate the practice of the present invention but are not intended to be limiting thereof.

### EXAMPLE 1

### Thermally Sensitive/Difficult to Process/Elastomer Blend

A 50/30/20 blend (by weight) is prepared from a polycaprolactone (TONE P787), a Biopol polyhydroxybutyrate/valerate copolymer (12% valerate content), and a polyurethane elastomer (Morthane PN3429-100) using the following procedure:

Along with 6810 grams Tone P787 pellets, 4086 grams Biopol and 2724 grams Morthane PN3429-100 granules are dry blended in a Kelly Duplex mixer for 15 minutes. The dry blend is melt compounded in a Rheomix TW-100 twin screw extruder with conical barrels and two partially intermeshing counterrotating screws and equipped with a horizontal rod die having a 0.125 inch (0.3175 cm) diameter nozzle. The temperature of the twin screw extruder varies from 275°F (135°C) in the first heating zone, 310°F (154°C) in the second zone, and 340°F (171°C) in the third zone at the discharge end near the die. The screw speed is maintained at 25 rpm. The molten strand is cooled and solidified in a water bath prior to entering a Berlyn Model PEL-2 pelletizer where it is chopped into pellets approximately 0.125 inches (0.3175 cm) long. The cooled pellets are then dried in a vacuum oven at 50°C prior to film processing.

A cast film is produced from the compounded pellets using a Rheomix Model 202 0.75 inch (1.905 cm) diameter single screw extruder equipped with a 6 inch (15.24 cm) wide horizontal sheet die utilizing a 0.04 inch (0.1016 cm) die gap. A constant taper screw having a 20:1 length to diameter ratio and 3:1 compression ratio is employed. The temperature of the first heating zone is maintained at 370°F (188°C), the second heating zone is maintained at 380°F (193°C), and the die is maintained at 320°F (160°C). Screw speed is maintained at 25 rpm. The molten film passes from the die to a Postex sheet take-off system where it is cooled to room temperature and then collected on a cardboard core. Take-off speed is adjusted to provide a film about 4 inches (10.16 cm) wide and about 0.001 inch (25.4 microns) thick.

The resulting film is translucent, and displays properties outlined below:
a) a machine direction (MD) tensile modulus of 6.84x10⁹ dynes/sq. cm. (99,200 psi),
b) a MD tear strength of 99 grams per 25.4 microns of thickness,
c) a cross direction tear strength of 199 grams per 25.4 microns of thickness,
d) an impact strength of 77 cm,
e) a moisture transport rate of 0.00048 grams per sq. cm. per 16 hours,
f) a modulus at 60°C of 1.4x10⁹ dynes/sq. cm. (20,300 psi)

As can be seen, the properties of the film meet the aforementioned specifications for backsheet films of absorbent articles.

### EXAMPLE 2

### Thermally Sensitive/Elastomer Blend

A 60/40 (by weight) composition is prepared by initially dry blending 8178 grams of Tone P787, and 5448 grams Morthane PN3429-100 as outlined in Example 1. The melt compounding and film extrusion procedures of Example 1 are followed to provide a film with properties as outlined below.
a) a machine direction (MD) tensile modulus of 2.16x10⁹ dynes/sq. cm. (31,300 psi),
b) a MD tear strength of 142 grams per 25.4 microns of thickness,
c) a cross direction tear strength of 289 grams per 25.4 microns of thickness,
d) an impact strength of 128 cm,
e) a moisture transport rate of 0.0006 grams per sq. cm per 16 hours, and
f) a modulus at 60°C of 6.9x10⁷ dynes/sq. cm. (1000 psi).

As can be seen, the properties of the film meet the afore-mentioned specifications for backsheet films of absorbent articles.

## Claims

1. A monolayer backsheet film having
a) biodegradability;
b) liquid-impermeability;
c) a machine direction (MD) tensile modulus from 6.895 x 10⁸ dynes/sq. cm. to 6.895 x 10⁹ dynes/sq. cm.;
d) a MD tear strength of at least 70 grams per 25,4 microns of thickness;
e) a cross machine direction (CD) tear strength of at least 70 grams per 25.4 microns of thickness;
f) an impact strength of at least 12 centimeters as measured by falling ball drop;
g) a moisture transport rate less than 0.0012 grams per square centimeter per 16 hours;
h) a modulus at 60°C of at least 5.52 x 10⁷ dynes/sq. cm.; and
i) a thickness from 12 microns to 75 microns;
said film comprising two or more polymers selected from the group consisting of:
a) moisture sensitive polymers, preferably thermoplastic polyvinyl alcohol compositions or hydroxypropylcellulose;
b) thermally sensitive polymers, preferably polycaprolactone, other aliphatic polyesters having melting points below 65°C or Vicat softening points of 45°C or less, or mixtures thereof;
c) mechanically limited polymers, preferably cellulose esters, cellulose ester blends with polycaprolactone, cellulose ester blends with polyhydroxybutyrate/valerate copolymers, or cellulose ester blends with thermally sensitive aliphatic polyesters;
d) polymers difficult to process into films, preferably polyhydroxybutyrate, polyhydroxybutyrate/valerate copolymers, or mixtures thereof;
e) hydrolytically cleavable aromatic/aliphatic polyester copolymers, preferably copolymers of polyethylene terephthalate or polybutylene terephthalate wherein the aliphatic fraction of said copolymers is derived from the group consisting of oxalates, malonates, succinates, glutarates, adipates, pimelates, suberates, azelotes, sobacates, nonanedinates, glycolates, and mixtures thereof;
f) oxidized ethylene/carbon monoxide copolymers, preferably derived from ethylene/monoxide copolymers containing 0.5 to 20 weight percent carbon monoxide;
g) high melting aliphatic polyesters, preferably aliphatic polyesters having melting temperatures or glass transition temperatures above 65°C; and
h) elastomers, preferably polycaprolactone/diene block copolymers or aliphatic polyester urethanes,
said film being characterized by comprising either
- 1-40 weight percent of a moisture sensitive polymer, preferably a thermoplastic polyvinyl alcohol composition; and
- 99-60 weight percent of a second polymer selected from the group consisting of hydrolytically cleavable aromatic/aliphatic polyester copolymers, oxidized ethylene/carbon monoxide copolymers, and high melting aliphatic polyesters;
or said film being characterized by comprising
- 30-70 weight percent of a mechanically limited polymer, preferably a cellulose ester; and
- 70-30 weight percent of an elastomer, preferably an aliphatic polyester urethane.

2. The film of Claim 1 wherein the film comprises a blend of three polymers selected from the group consisting of: moisture sensitive polymers, preferably a thermoplastic polyvinyl alcohol composition; thermally sensitive polymers, preferably polycaprolactone: elastomers, preferably an aliphatic polyester urethane; and mechanically limited polymers, preferably a cellulose ester.

3. The film of Claim 1 wherein the film comprises a blend of three polymers selected from the group consisting of hydrolytically cleavable aromatic/aliphatic polyester copolymers; thermally sensitive polymers, preferably polycaprolactone; and moisture sensitive copolymers, preferably a thermoplastic polyvinyl alcohol composition.

4. The film of Claim 1 wherein the film comprises a blend of three polymers selected from the group consisting of: oxidized ethylene/carbon monoxide copolymers; thermally sensitive polymers, preferably polycaprolactone; and moisture sensitive polymers, preferably a thermoplastic polyvinyl alcohol composition.

5. The film of Claim 1 wherein the film comprises a blend of three polymers selected from the group consisting of thermally sensitive polymers, preferably polycaprolactone; polymers difficult to process into films, preferably a polyhydroxybutyrate/valorate copolymer, and elastomers, preferably an aliphatic polyester urethane.

## Patentansprüche

1. Eine einschichtige Rückenblattfolie, welche aufweist
a) biologische Abbaubarkeit;
b) Flüssigkeitsundurchlässigkeit;
c) einen Zugmodul in Maschinenrichtung [MD] von 6,895 x 10⁸ dyn/cm² bis 6,895 x 10⁹ dyn/cm²;
d) eine MD Reißfestigkeit von mindestens 70 Gramm pro 25,4 Mikron Dicke;
e) eine Reißfestigkeit quer zur Maschinenrichtung [CD] von mindestens 70 Gramm pro 25,4 Mikron Dicke;
f) eine Stoßfestigkeit von mindestens 12 cm, gemessen durch die Kugelfallmethode;
g) eine Feuchtigkeitstransportgeschwindigkeit von weniger als 0,0012 g/cm²pro 16 Stunden;
h) einen Modul bei 60°C von mindestens 5,52 x 10⁷ dyn/cm²; und
i) eine Dicke von 12 Mikron bis 75 Mikron;
wobei diese Folie zwei oder mehr Polymere umfaßt, die ausgewählt sind aus der Gruppe, die besteht aus:
a) feuchtigkeitsempfindlichen Polymeren, vorzugsweise thermoplastischen Polyvinylalkohol-Zusammensetzungen oder Hydroxypropylzellulose;
b) thermisch empfindlichen Polymeren, vorzugsweise Polycaprolacton, anderen aliphatischen Polyestern mit Schmelzpunkten unter 65°C oder Vicat-Erweichungspunkten von 45°C oder weniger oder Mischungen hievon;
c) mechanisch begrenzten Polymeren, vorzugsweise Zelluloseestern, Zelluloseester-Mischungen mit Polycaprolacton, Zelluloseester-Mischungen mit Polyhydroxybutyrat/valerat-Copolymeren oder Zelluloseester-Mischungen mit thermisch empfindlichen aliphatischen Polyestern;
d) Polymeren, die schwer zu Folien zu verarbeiten sind, vorzugsweise Polyhydroxybutyrat, Polyhydroxybutyrat/valerat-Copolymeren oder Mischungen hievon;
e) hydrolytisch spaltbaren aromatisch/aliphatischen Polyester-Copolymeren, vorzugsweise Copolymeren von Polyethylenterephthalat oder Polybutylenterephthalat, worin der aliphatische Anteil der genannten Copolymeren von der aus Oxalaten, Malonaten, Succinaten, Glutaraten, Adipaten, Pimelaten, Suberaten, Azelaten, Sebacaten, Nonandinaten, Glycolaten und Mischungen hievon bestehenden Gruppe abgeleitet ist;
f) oxidierten Ethylen/Kohlenmonoxid-Copolymeren, die vorzugsweise von Ethylen/Monoxid-Copolymeren abgeleitet sind, die 0,5 bis 20 Gew.-% Kohlenmonoxid enthalten;
g) hochschmelzenden aliphatischen Polyestern, vorzugsweise aliphatischen Polyestern mit Schmelztemperaturen oder Glasübergangstemperaturen über 65°C; und
h) Elastomeren, vorzugsweise Polycaprolacton/Dien-Blockcopolymeren oder aliphatischen Polyesterurethanen,
wobei die genannte Folie entweder dadurch gekennzeichnet ist, daß sie
zu 1 - 40 Gew.-% ein feuchtigkeitsempfindliches Polymer, vorzugsweise eine thermoplastische Polvvinylalkohol-Zusammensetzung; und
zu 99 - 60 Gew.-% ein zweites Polymer, das aus der aus hydrolytisch spaltbaren aromatisch/aliphatischen Polyester-Copolymeren, oxidierten Ethylen/Kohlenmonoxid-Copolymeren und hochschmelzenden aliphatischen Polyestern bestehenden Gruppe ausgewählt ist,
enthält,
oder die genannte Folie dadurch gekennzeichnet ist, daß sie
zu 30-70 Gew.-% ein mechanisch begrenztes Polymer, vorzugsweise einen Zelluloseester; und
zu 70-30 Gew.-% ein Elastomer, vorzugsweise ein aliphatisches Polyesterurethan,
enthält.

2. Die Folie nach Anspruch 1, worin die Folie eine Mischung aus drei Polymeren umfaßt, die aus der aus feuchtigkeitsempfindlichen Polymeren, vorzugsweise einer thermoplastischen Polyvinylalkohol-Zusammensetzung; thermisch empfindlichen Polymeren, vorzugsweise Polycaprolacton; Elastomeren, vorzugsweise einem aliphatischen Polyesterurethan; und mechanisch begrenzten Polymeren, vorzugsweise einem Zelluloseester, bestehenden Gruppe ausgewählt sind.

3. Die Folie nach Anspruch 1, worin die Folie eine Mischung aus drei Polymeren umfaßt, die aus der aus hydrolytisch spaltbaren aromatisch/aliphatischen Polyester-Copolymeren; thermisch empfindlichen Polymeren, vorzugsweise Polycaprolacton; und feuchtigkeitsempfindlichen Copolymeren, vorzugsweise einer thermoplastischen Polyvinylalkohol-Zusammensetzung, bestehenden Gruppe ausgewählt sind.

4. Die Folie nach Anspruch 1, worin die Folie eine Mischung aus drei Polymeren umfaßt, die aus der aus oxidierten Ethylen/Kohlenmonoxid-Copolymeren; thermisch empfindlichen Polymeren, vorzugsweise Polycaprolacton; und feuchtigkeitsempfindlichen Polymeren, vorzugsweise einer thermoplastischen Polyvinylalkohol-Zusammensetzung, bestehenden Gruppe ausgewählt sind.

5. Die Folie nach Anspruch 1, worin die Folie eine Mischung aus drei Polymeren umfaßt, die aus der aus thermisch empfindlichen Polymeren, vorzugsweise Polycaprolacton; Polymeren, die schwer zu Folien zu verarbeiten sind, vorzugsweise einem Polyhydroxybutyrat/valerat-Copolymer, und Elastomeren, vorzugsweise einem aliphatischen Polyesterurethan, bestehenden Gruppe ausgewählt sind.

## Revendications

1. Film de feuille de fond monocouche possédant:
a) une biodégradabilité;
b) une imperméabilité aux liquides;
c) un module de traction dans le sens machine (SM) de 6,895 x 10⁸ dynes/cm² à 6,895 x 10⁹ dynes/cm²;
d) une résistance à la déchirure SM d'au moins 70 grammes par 25,4 microns d'épaisseur;
e) une résistance à la déchirure dans le sens travers (ST) d'au moins 70 grammes par 25,4 microns d'épaisseur;
f) une résistance au choc d'au moins 12 centimètres, mesurée par la chute d'une bille tombante;
g) une vitesse de transport de l'humidité inférieure à 0,0012 grammes par centimètre carré pendant 16 heures;
h) un module à 60°C d'au moins 5,52 x 10⁷ dynes/cm²; et
i) une épaisseur de 12 microns à 75 microns;
ledit film comprenant deux polymères ou plus, choisis dans le groupe constitué par:
a) les polymères sensibles à l'humidité, de préférence des compositions de poly(alcool vinylique) thermoplastiques ou d'hydroxypropylcellulose;
b) les polymères sensibles à la chaleur, de préférence une polycaprolactone, d'autres polyesters aliphatiques ayant des points de fusion inférieurs à 65°C ou des points de ramollissement Vicat de 45°C ou moins, ou des mélanges de ceux-ci;
c) les polymères limités mécaniquement, de préférence des esters de cellulose, des mélanges d'esters de cellulose avec une polycaprolactone, des mélanges d'esters de cellulose avec des copolymères polyhydroxybutyrate/valérate, ou des mélanges d'esters de cellulose avec des polyesters aliphatiques sensibles à la chaleur;
d) les polymères difficiles à transformer en films, de préférence un polyhydroxybutyrate, des copolymères polyhydroxybutyrate/valérate, ou des mélanges de ceux-ci;
e) des copolymères de polyesters aromatiques/aliphatiques susceptibles d'être coupés par hydrolyse, de préférence des copolymères de poly(éthylène-téréphtalate) ou de poly(butylène-téréphtalate) dans lesquels la fraction aliphatique desdits copolymères provient du groupe constitué par les oxalates, les malonates, les succinates, les glutarates, les adipates, les pimélates, les subérates, les azélates, les sébaçates, les nonanedioates, les glycolates, et leurs mélanges;
f) les copolymères oxyde d'éthylène/monoxyde de carbone, de préférence dérivés de copolymère éthylène/monoxyde contenant 0,5 à 20 pour cent en poids de monoxyde de carbone;
g) les polyesters aliphatiques à point de fasion élevé, de préférence les polyesters aliphatiques ayant des températures de fusion ou des températures de transition vitreuse supérieures à 65°C; et
h) les élastomères, de préférence les copolymères séquencés polycaprolactone/diène ou les polyester-uréthanes aliphatiques,
ledit film étant soit caractérisé en ce qu'il comprend
- 1-40 pour cent en poids d'un polymère sensible à l'humidité, de préférence une composition de poly(alcool vinylique) thermoplastique; et
- 99-60 pour cent en poids d'un second polymère choisi dans le groupe constitué par les copolymères polyesters aromatiques/aliphatiques susceptibles d'être coupés par hydrolyse, les copolymères oxyde d'éthylène/monoxyde de carbone et les polyesters aliphatiques à point de fusion élevé;
soit caractérisé en ce qu'il comprend:
- 30-70 pour cent en poids d'un polymère limité mécaniquement, de préférence un ester de cellulose; et
- 70-30 pour cent en poids d'un élastomère, de préférence d'un polyester-uréthane aliphatique.

2. Film selon la revendication 1, caractérisé en ce que le film comprend un mélange de trois polymères choisis dans le groupe constitué par: les polymères sensibles à l'humidité, de préférence une composition de poly(alcool vinylique) thermoplastique; les polymères sensibles à la chaleur, de préférence une polycaprolactone; les élastomères, de préférence un polyester-uréthane aliphatique; et les polymères limités mécaniquement, de préférence un ester de cellulose.

3. Film selon la revendication 1, caractérisé en ce que le film comprend un mélange de trois polymères choisis dans le groupe constitué par les copolymères de polyesters aromatiques/aliphatiques susceptibles d'être coupés par hydrolyse; les polymères sensibles à la chaleur, de préférence une polycaprolactone; et les copolymères sensibles à l'humidité, de préférence une composition de poly(alcool vinylique) thermoplastique.

4. Film selon la revendication 1, caractérisé en ce que le film comprend un mélange de trois polymères choisis dans le groupe constitué par les copolymères oxyde d'éthylène/monoxyde de carbone; les polymères sensibles à la chaleur, de préférence une polycaprolactone; et les polymères sensibles à l'humidité, de préférence une composition de poly(alcool vinylique) thermoplastique.

5. Film selon la revendication 1, caractérisé en ce que le film comprend un mélange de trois polymères choisis dans le groupe constitué par les polymères sensibles à la chaleur, de préférence une polycaprolactone; les polymères difficiles à transformer en films, de préférence un copolymère polyhydroxybutyrate/valérate; et les élastomères, de préférence un polyester-uréthane aliphatique.
